# EUROPEAN PATENT APPLICATION

(11) **EP 4 386 086 A1**
(43) Date of publication of application: **19.06.2024**
(21) Application number: 22856239.3
(22) Date of filing: 11.08.2022
(51) Int. Cl.: C12N 13/00, C12N 5/077

(54) **COMPOSITION FOR TREATMENT OF CARTILAGE-RELATED DISEASE AND PREPARATION METHOD THEREFOR**

(30) Priority: 12.08.2021 KR 20210106922; 30.09.2021 KR 20210130412
(71) Applicant: Youth Bio Global Co., Ltd., Seoul 08381 (KR)
(72) Inventor: YOO, Seung Ho, Seoul 05607 (KR); CHANG, Chi Young, Seoul 08223 (KR)
(74) Representative: Plasseraud IP
(86) International application number: PCT/KR2022/011985
(87) International publication number: WO 2023/018244

(57) **Abstract**

The present disclosure relates to a pharmaceutical composition for treatment of a cartilage-related disease prepared by applying an electric stimulus and a method for preparing the same. Specifically, the composition of the present disclosure can be prepared by applying only an electric stimulus to a stem cell aggregate without introducing externally derived growth factors, etc. The composition has the advantage of remarkably reducing medical expenses because it can be prepared without using expensive growth factors. In addition, the composition for treatment of a cartilage-related disease according to the present disclosure does not express COL2, which is a mature chondrocyte marker, as compared to the conventional cells prepared for treating a cartilage-related disease and, thus, has the advantage of less immune-related side effects than mature cells.

## Description

### Technical Field]

The present disclosure relates to a pharmaceutical composition for treatment of a cartilage-related disease prepared by applying an electric stimulus and a method for preparing the same.

### [Background Art]

Articular cartilage damage is a very common problem that affects millions of people. Since the articular cartilage tissue is avascular and lacks stem cells, the ability of cartilage regeneration in adults is limited. Defects that extend to the subchondral bone induce the formation of fibrous or fibrocartilaginous tissue and the restored tissue, which is biochemically and biomechanically from hyaline cartilage, undergoes immature degeneration. A problem in the joint necessarily leads to pain or severe restriction in movement. In the past, there was no proper treatment and there was no significant difference between joint age and actual age due to relatively shorter average lifespan than today. However, at present, lifespan has increased but the joint lifespan fails to catch up the actual lifespan due to various environmental factors. The excessive use of the knee causes wearing out of the cartilage and leads to degenerative arthritis, which affects 8 out of 10 people aged 65 years or older in Korea, as a result of inflammation in the knee joint and ligament. Degenerative arthritis, which has been regarded as a geriatric disease, is now a factor that causes arthritis in young people because of incorrect lifestyle habits, increased traumatic arthritis due to excessive exercise, etc.

To treat the cartilage-related diseases, methods of using artificial cartilage containing chondrocytes isolated from the costal cartilage (Korean Patent Application No. 10-2006-0106812) or using stem cells, etc. are being studied. However, the methods of using stem cells are costly to be utilized clinically because they use expensive growth factors for differentiation into chondrocytes. Because the problems of cost, immunity, etc. have not been resolved yet, there is a need to develop a therapeutic agent for treating a cartilage-related disease that solves these problems.

### [Disclosure]

### [Technical Problem]

Under this background, the inventors of the present disclosure have studied and made efforts to develop a therapeutic agent that can treat a cartilage-related disease without addition of growth factors. As a result, they have found out that stem cells can be differentiated into chondroprogenitor cells that have the characteristics of chondrocytes without expressing the mature chondrocyte marker COL2 by applying an electric stimulus and have completed the present disclosure.

The present disclosure is directed to providing a pharmaceutical composition for treatment or prevention of a cartilage-related disease, which contains chondroprogenitor cells having the following characteristics or an aggregate thereof as an active ingredient: (a) not expressing COL2; and (b) the chondroprogenitor cells being substrate-stained by one or more selected from a group consisting of alcian blue, safranin O and toluidine blue.

The present disclosure is also directed to providing a method for preparing a pharmaceutical composition for treatment or prevention of a cartilage-related disease, which includes a step of preparing chondroprogenitor cells or an aggregate thereof by applying an electric stimulus to stem cells, wherein the chondroprogenitor cells have the following characteristics: (a) not expressing COL2; and (b) the chondroprogenitor cells being substrate-stained by one or more selected from a group consisting of alcian blue, safranin O and toluidine blue.

The present disclosure is also directed to providing a method for treatment or prevention of a cartilage-related disease, which includes a step of administering chondroprogenitor cells having the following characteristics or an aggregate thereof to a subject in need thereof: (a) not expressing COL2; and (b) the chondroprogenitor cells being substrate-stained by one or more selected from a group consisting of alcian blue, safranin O and toluidine blue.

The present disclosure is also directed to providing a use of chondroprogenitor cells having the following characteristics or an aggregate thereof for preparing a medicine for treatment or prevention of a cartilage-related disease: (a) not expressing COL2; and (b) the chondroprogenitor cells being substrate-stained by one or more selected from a group consisting of alcian blue, safranin O and toluidine blue.

However, the technical problems to be solved by the present disclosure are not limited to those mentioned above and other problems not mentioned above will be clearly understood by those skilled in the art from the following description.

### [Technical Solution]

The present disclosure provides a pharmaceutical composition for treatment or prevention of a cartilage-related disease, which contains chondroprogenitor cells having the following characteristics or an aggregate thereof as an active ingredient:
(a) not expressing COL2; and
(b) the chondroprogenitor cells being substrate-stained by one or more selected from a group consisting of alcian blue, safranin O and toluidine blue.

In an exemplary embodiment of the present disclosure, the cartilage-related disease may be selected from a group consisting of osteoarthritis, arthritis, meniscus derangement, rheumatoid arthritis, tear of meniscus, triangular fibrocartilage complex tear, traumatic cartilage damage and degenerative arthritis.

In another exemplary embodiment of the present disclosure, the active ingredient of the pharmaceutical composition may contain 90% or more cells homogeneous with the chondroprogenitor cells.

In another exemplary embodiment of the present disclosure, the chondroprogenitor cells may be induced to differentiate from stem cells.

In another exemplary embodiment of the present disclosure, the stem cells may be mesenchymal stem cells.

In another exemplary embodiment of the present disclosure, the induction of differentiation may be achieved by an electric stimulus.

In another exemplary embodiment of the present disclosure, the electric stimulus may have a frequency of 0 to 20 Hz; an amplitude of -20 to 20 V; and a duty ratio of 0 to 80%.

In another exemplary embodiment of the present disclosure, the chondroprogenitor cells may have a decreased expression level of one or more gene selected from a group consisting of COL1 and COL5 or a protein encoded by the gene as compared to mesenchymal stem cells; and an increased expression level of the COL6 gene or a protein encoded by the gene as compared to mesenchymal stem cells.

In another exemplary embodiment of the present disclosure, the chondroprogenitor cells may have an increased expression level of one or more gene selected from a group consisting of GJB2, GJC1, PECAM1, CLDN2, CLDN7, CLDN10 and CLDN19 or a protein encoded by the gene as compared to mesenchymal stem cells.

In another exemplary embodiment of the present disclosure, the pharmaceutical composition may be in a dosage form that is easy to be inserted directly into the cartilage.

In another exemplary embodiment of the present disclosure, the aggregate of the chondroprogenitor cells may be in a spheroid form.

In another exemplary embodiment of the present disclosure, the spheroid may have a diameter of 0.5-1.5 mm.

The present disclosure also provides a method for preparing a pharmaceutical composition for treatment or prevention of a cartilage-related disease, which includes a step of preparing chondroprogenitor cells or an aggregate thereof by applying an electric stimulus to stem cells, wherein the chondroprogenitor cells have the following characteristics: (a) not expressing COL2; and (b) the chondroprogenitor cells being substrate-stained by one or more selected from a group consisting of alcian blue, safranin O and toluidine blue.

The present disclosure also provides a method for treatment or prevention of a cartilage-related disease, which includes a step of administering chondroprogenitor cells having the following characteristics or an aggregate thereof to a subject in need thereof: (a) not expressing COL2; and (b) the chondroprogenitor cells being substrate-stained by one or more selected from a group consisting of alcian blue, safranin O and toluidine blue.

The present disclosure also provides a use of chondroprogenitor cells having the following characteristics or an aggregate thereof for preparing a medicine for treatment or prevention of a cartilage-related disease: (a) not expressing COL2; and (b) the chondroprogenitor cells being substrate-stained by one or more selected from a group consisting of alcian blue, safranin O and toluidine blue.

### [Advantageous Effects]

Since a composition for treatment of a cartilage-related disease according to the present disclosure can be prepared by applying only an electric stimulus to a stem cell aggregate without introducing externally derived growth factors, etc. and it can be prepared without using expensive growth factors, it has the advantage of remarkably reducing medical expenses. In addition, the composition for treatment of a cartilage-related disease according to the present disclosure does not express COL2, which is a mature chondrocyte marker, as compared to the conventional cells prepared for treating a cartilage-related disease and, thus, has the advantage of less immune-related side effects than mature cells.

However, the effects of the present disclosure are not limited to those described above and it should be understood that they include all the effects that can be inferred from the detailed description of the present disclosure or claims.

### [Brief Description of Drawings]

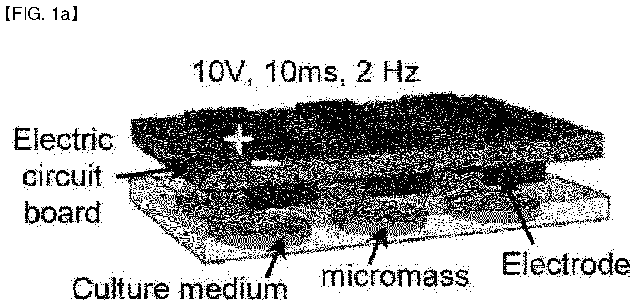

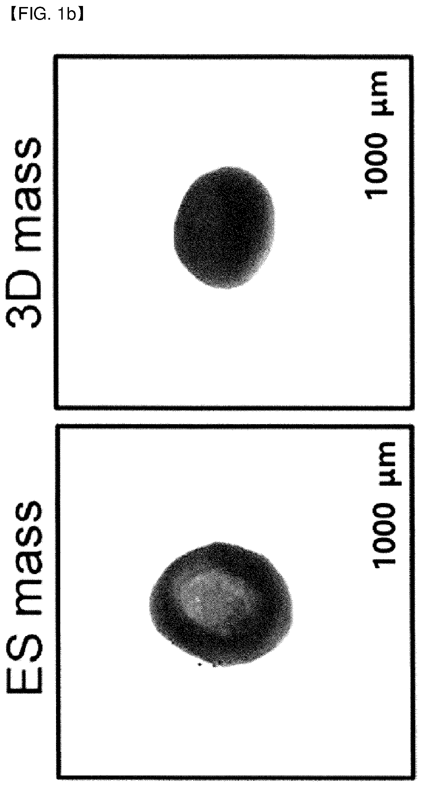

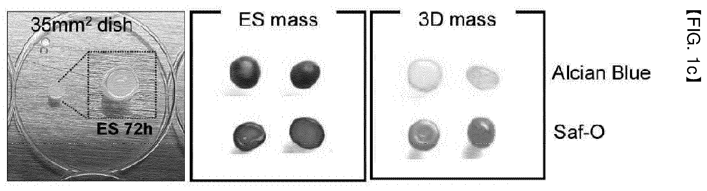

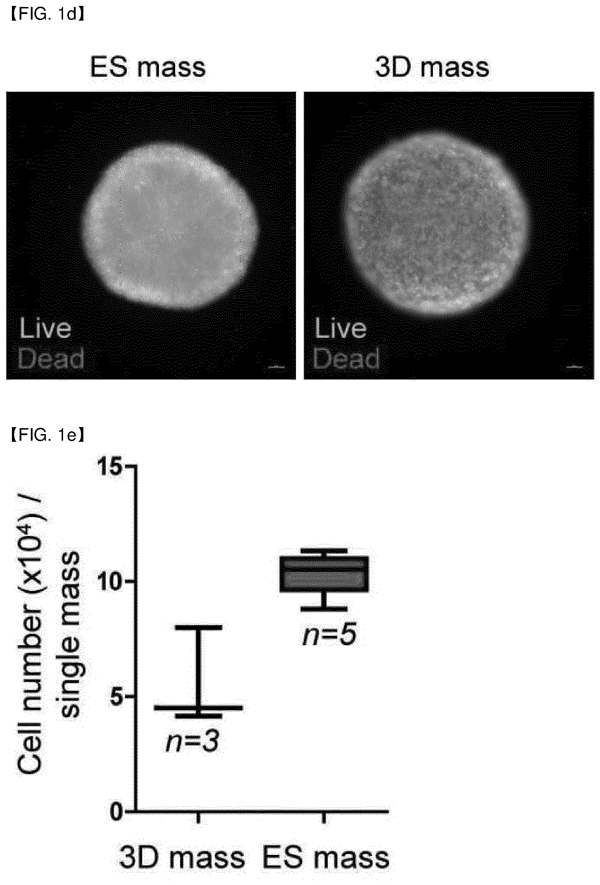

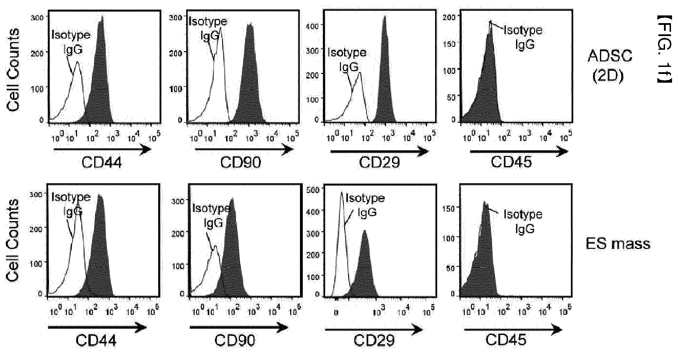

FIGS. 1a-1f describe application of an electric stimulus to cells. FIG. 1a schematically shows the condition of the electric stimulus and the application of the electric stimulus to cells, FIG. 1b shows a result of observing the cells to which the electric stimulus was applied with a phase-contrast microscope, FIG. 1c shows a result of observing the cells stained with alcian blue and safranin O, FIG. 1d shows a result of confirming cell viability with a Live/Dead viability cytotoxicity kit, FIG. 1e shows the cell number of a single micromass, and FIG. 1f shows a result of observing the level of membrane antigens of the cells.

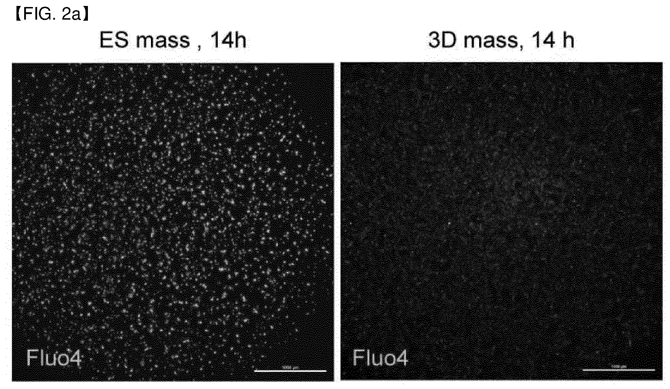

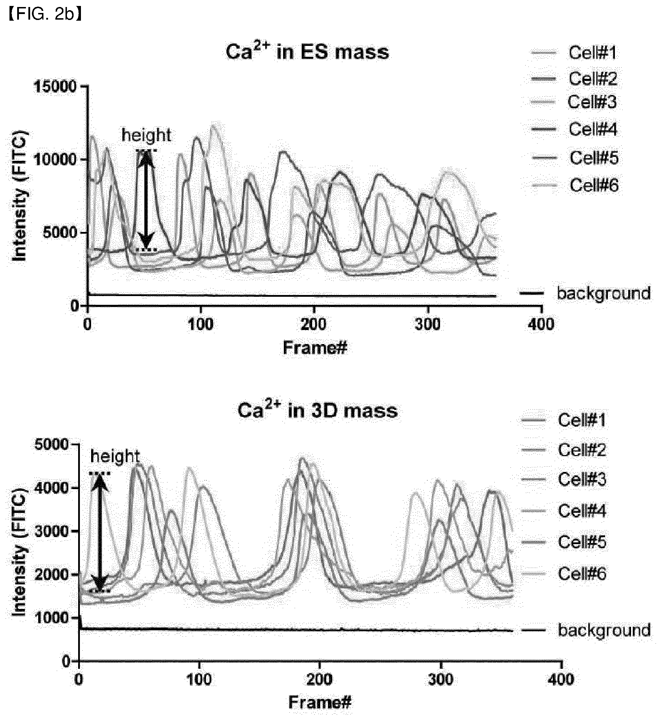

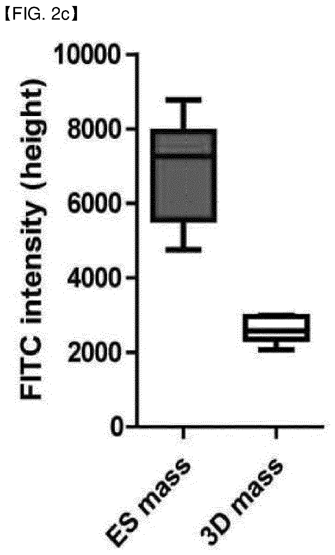

FIGS. 2a-2c show the calcium oscillation in cells by an electric stimulus. FIG. 2a shows a result of confirming the movement of calcium through Fluo-4 staining, FIG. 2b shows a result of confirming the concentration of Ca²⁺ in the cells over time, and FIG. 2c shows a result of investigating FITC intensity in the cells.

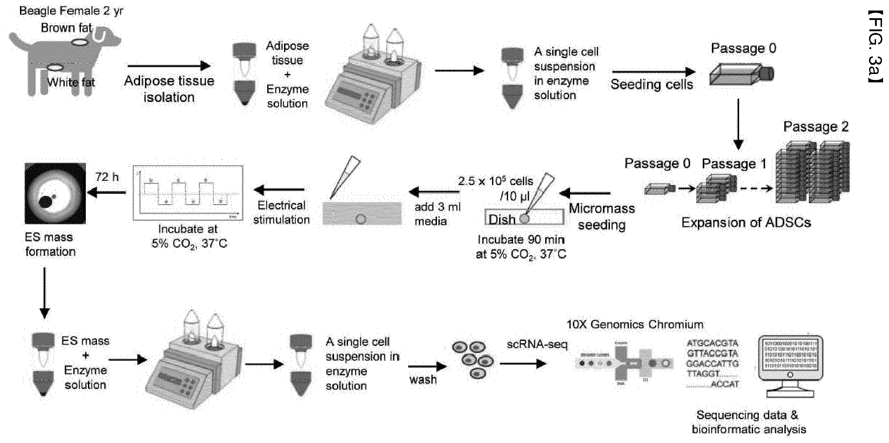

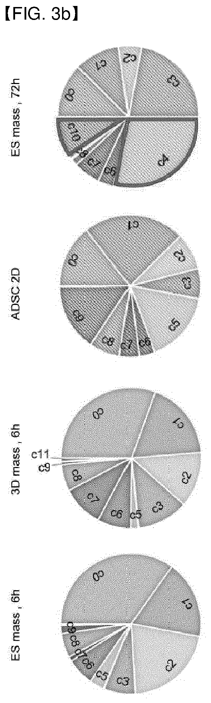

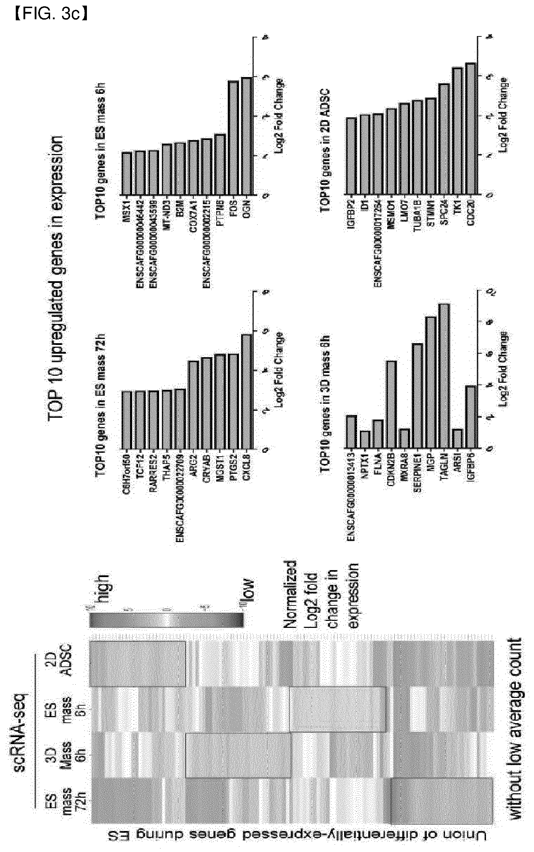

FIGS. 3a-3c show a result of investigating the change in gene expression caused by an electric stimulus. FIG. 3a schematically shows a workflow for single-cell RNA-seq analysis, FIG. 3b shows a result of clustering the gene expression profile of 2D-cultured ADSCs, a cell micromass to which an electric stimulus was not applied for 6 hours, a cell micromass to which an electric stimulus was applied for 6 hours and a cell micromass to which an electric stimulus was applied for 72 hours, and FIG. 3c is a heat map showing the expression of differentially expressed genes (DEGs) in four samples (Top 10 upregulated markers are shown on the right.).

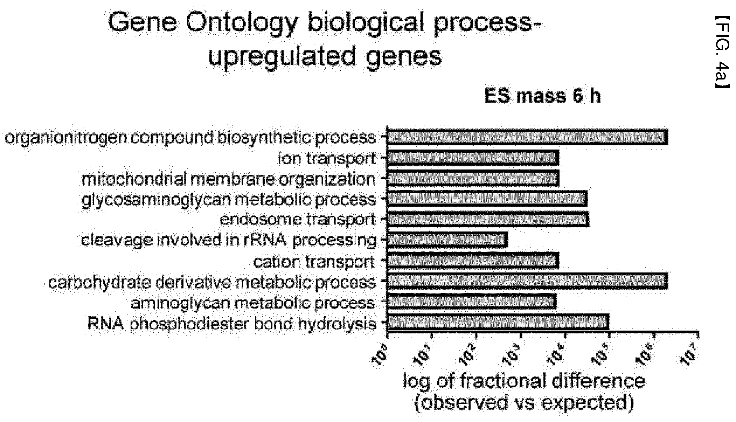

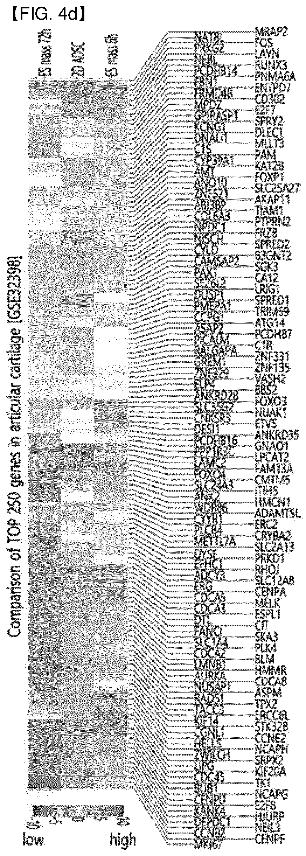

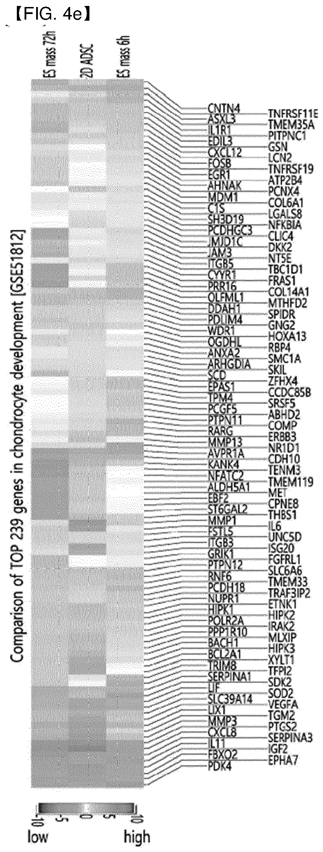

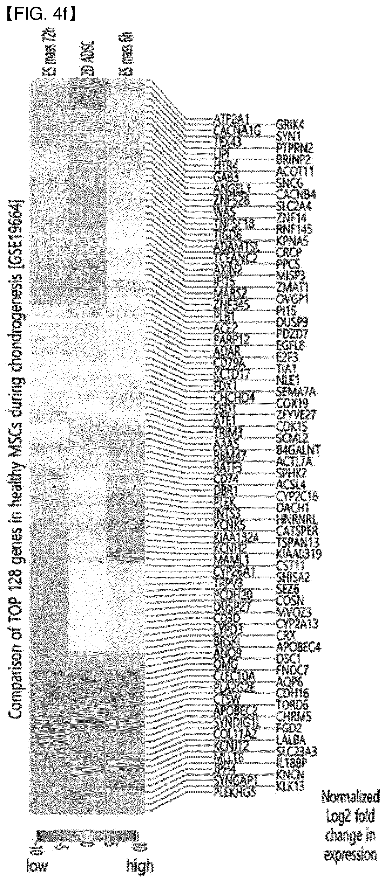

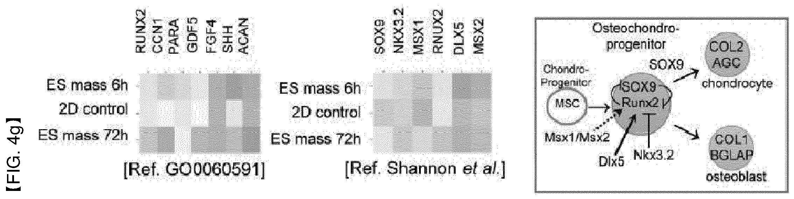

FIGS. 4a-4g show a result of investigating the gene expression of chondrogenesis markers in response to an electric stimulus. FIGS. 4a-4c show the result of gene ontology analysis for the top 20 genes that were distinctively upregulated in four samples, FIGS. 4d-f shows a result of comparing the gene expression profile of articular cartilage (left), developing chondrocytes (center) and BM-MSCs that differentiated into cartilage (right) using NCBI's GEO (Gene Expression Omnibus), and FIG. 4g shows a result of investigating the expression level of genes involved in chondrocyte development using the gene ontology data of GO0060591 chondroblasts.

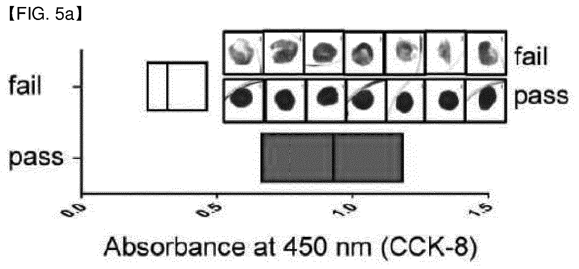

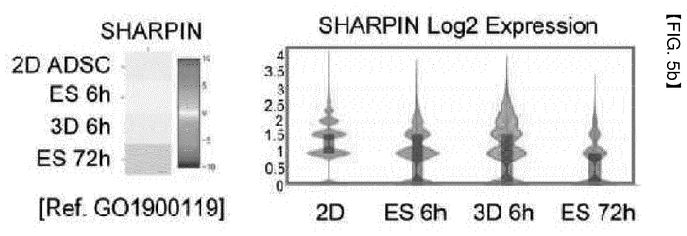

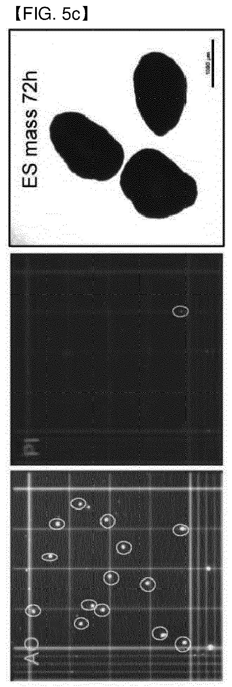

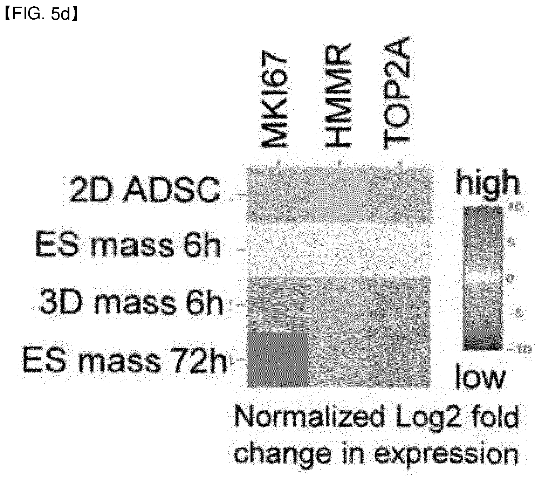

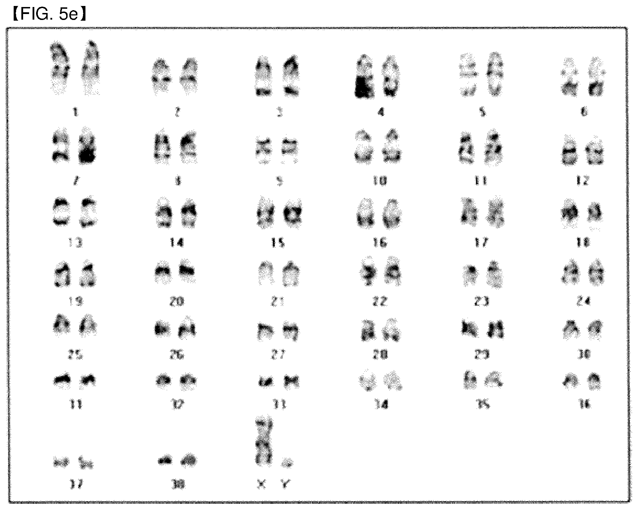

FIGS. 5a-5e show a result of investigating cell viability and change in cellular karyotype in response to an electric stimulus. FIG. 5a shows a result of transferring a condensed cell micromass and fragmented cells to a 96-well plate after applying an electric stimulus for 3 days and then investigating cell viability using a CCK-8 viability assay kit, FIG. 5b shows a result of investigating the mRNA expression level of SHARPIN, an apoptosis-related gene, for each cell sample to which the electric stimulus was applied, FIG. 5c shows a result of confirming the degree of cell death at the single cell level of the micromass through AO/PI (acridine orange/propidium iodide) staining, FIG. 5d shows the expression heat map of stem ness-related genes such as MKI67, HMMR, TOP2A, etc., and FIG. 5e shows a result of investigating normal karyotype in the cells of the micromass to which the electric stimulus was applied.

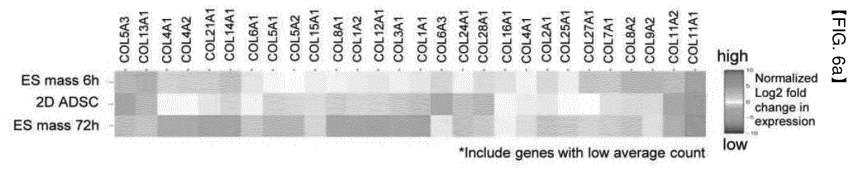

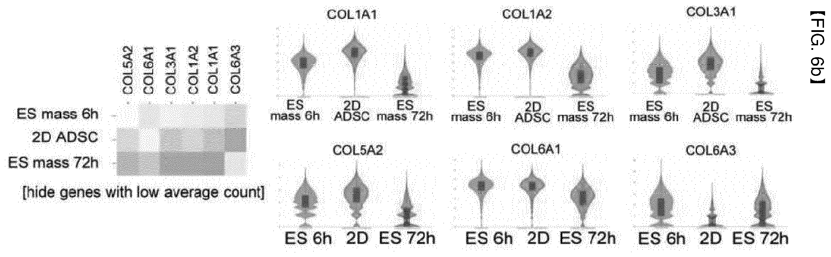

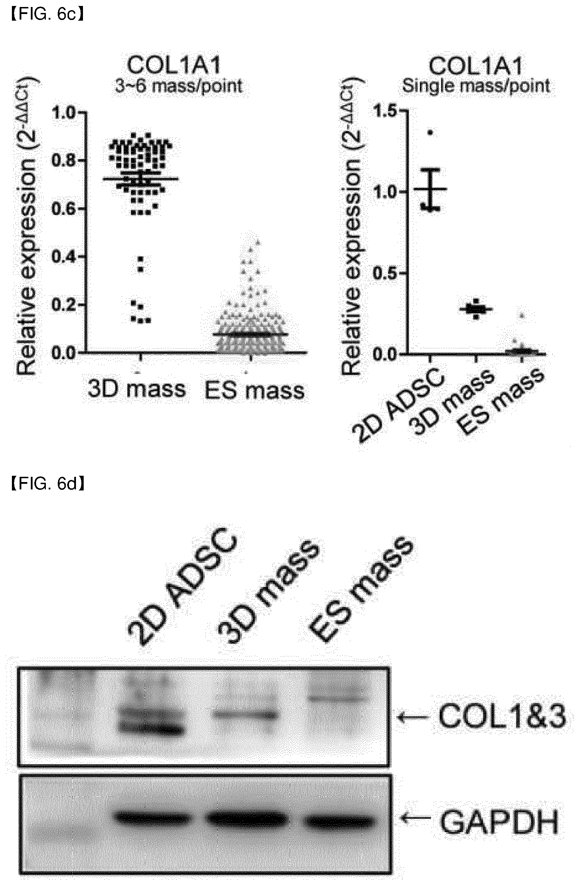

FIGS. 6a-6d show a result of investigating the change in collagen expression level in cells in response to the application of an electric stimulus. FIG. 6a shows the change in the expression level of all types of collagen in the cells, FIG. 6b shows a result of investigating the change in the expression of COL1A1, COL1A2, COL3A1, COL5A2, COL6A1 and COL6A3 that showed significant changes, FIG. 6c shows a result of investigating the expression level of COL1A1 by RT-PCR, and FIG. 6d shows a result of investigating the expression level of COL1A1 through western blotting.

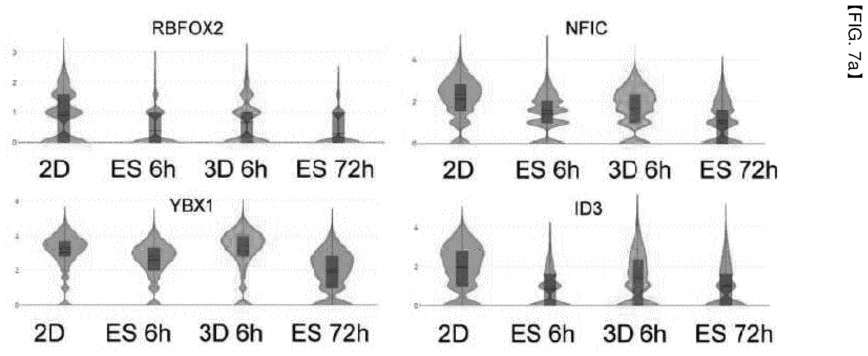

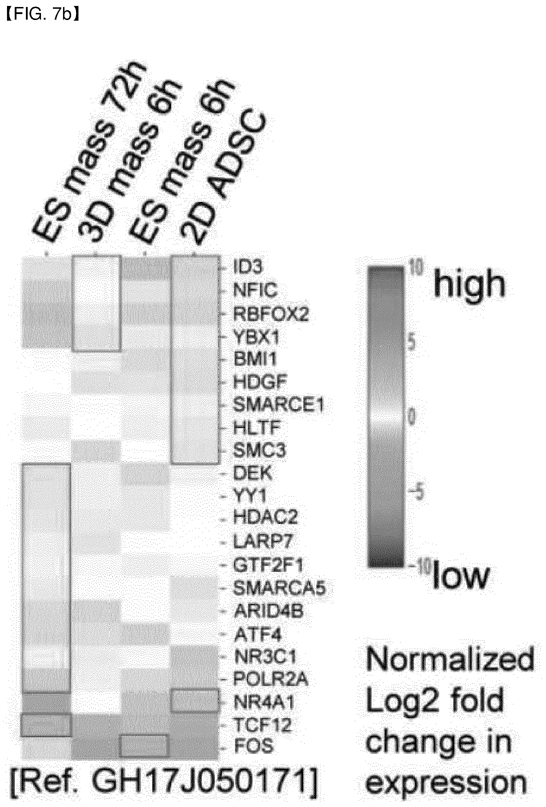

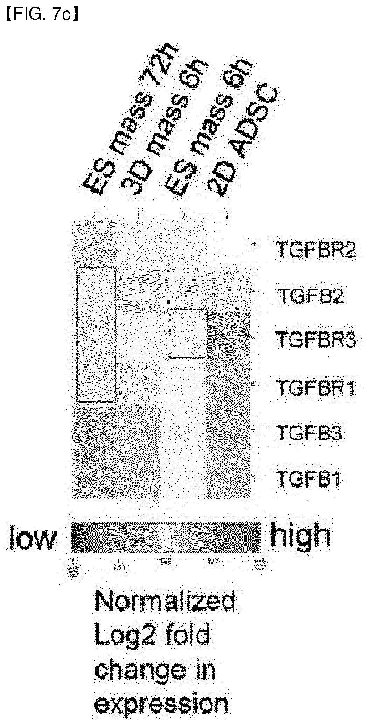

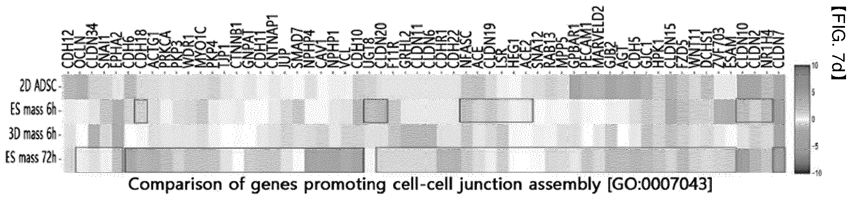

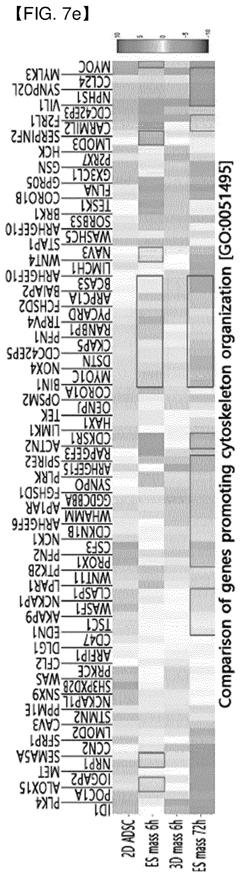

FIGS. 7a-7e show a result of investigating the regulation of the genetic expression of type 1 collagen in response to the application of an electric stimulus. FIG. 7a shows a result of investigating the expression of transcription factor genes such as RBFOX2, NFIC, YBX1 and ID3, which are related with TGFβ signaling, FIG. 7b shows a result of investigating the expression level of transcription factors binding to the promoter/enhancer of COL1A1, FIG. 7c is a heat map showing the expression level of the TGFβ superfamily and receptors thereof, and FIG. 7d and FIG. 7e show a result of investigating the change in the expression level of tissue formation-related genes GO0007043 (cell-cell junction assembly) and GO0051495 (positive regulation of cytoskeleton organization) in the cells to which the electric stimulus was applied using a gene ontology data portal.

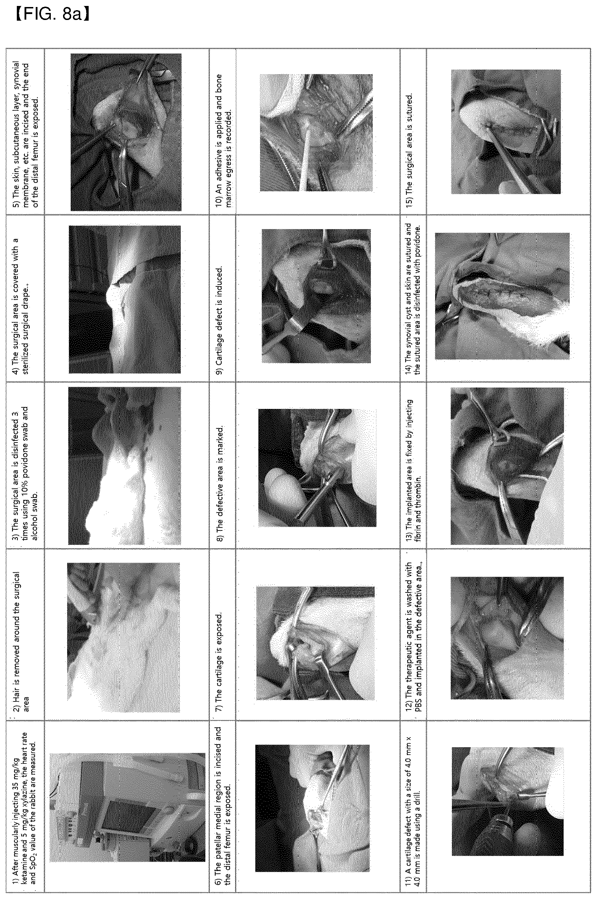

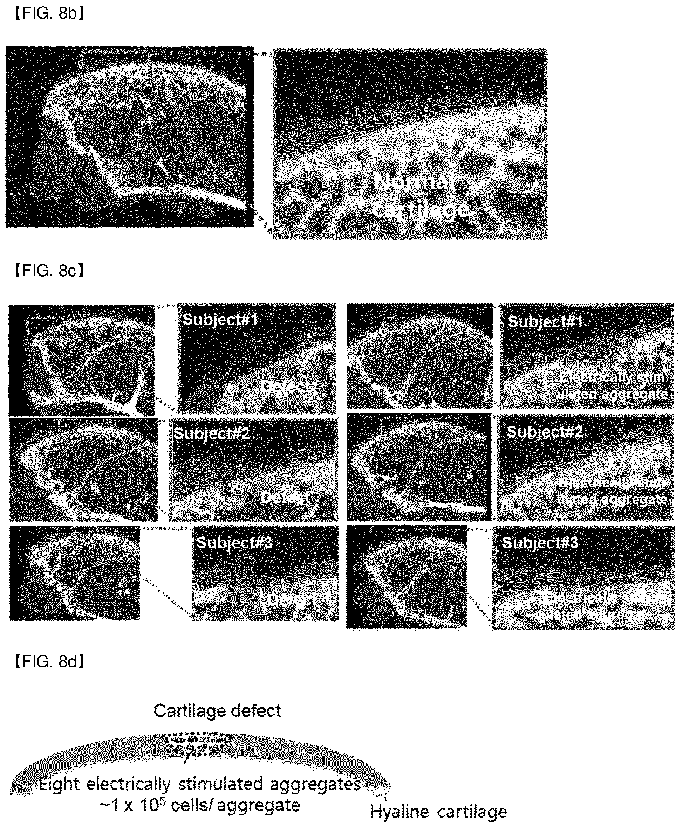

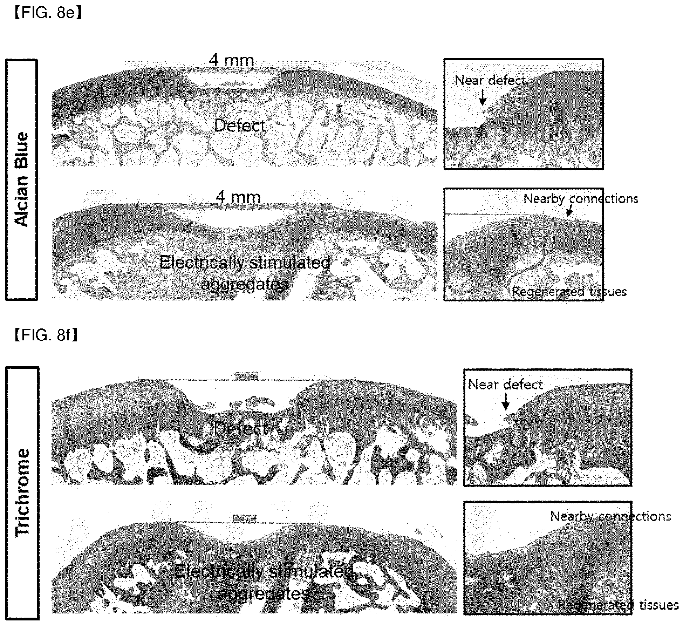

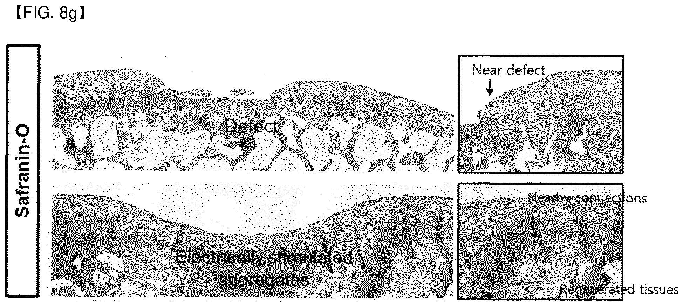

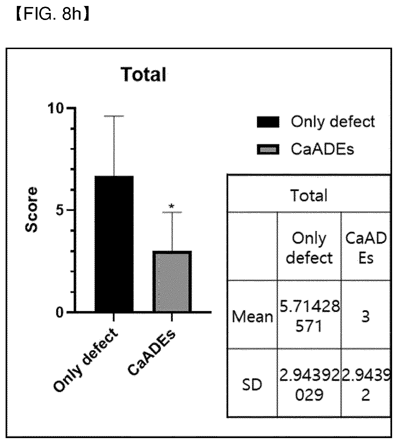

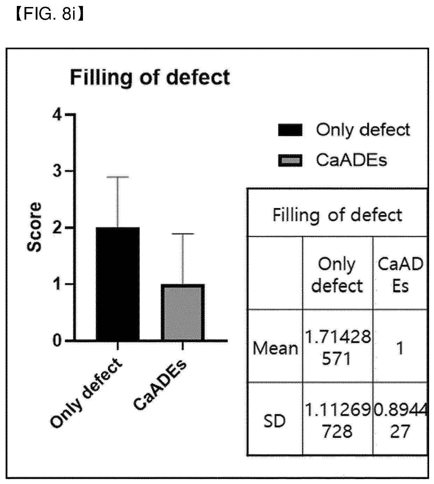

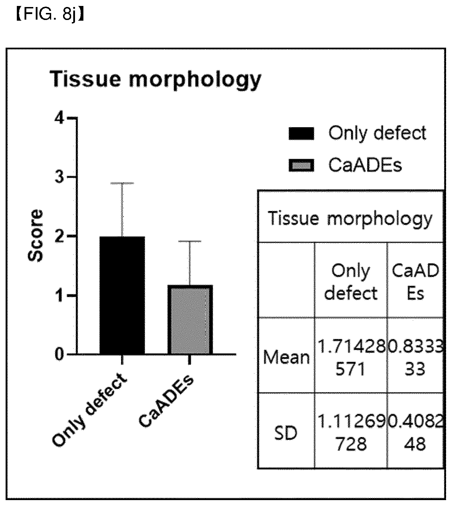

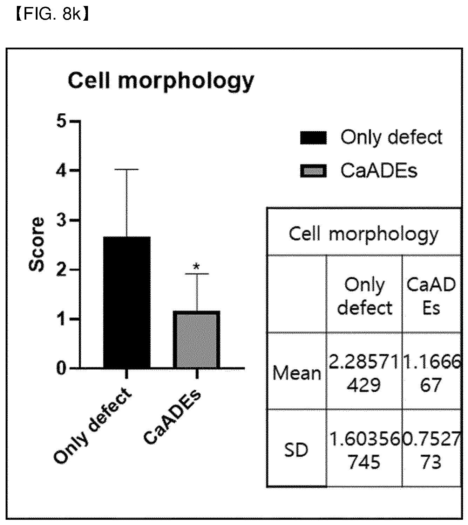

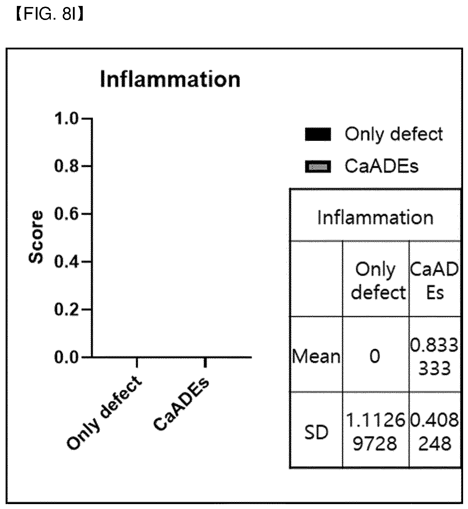

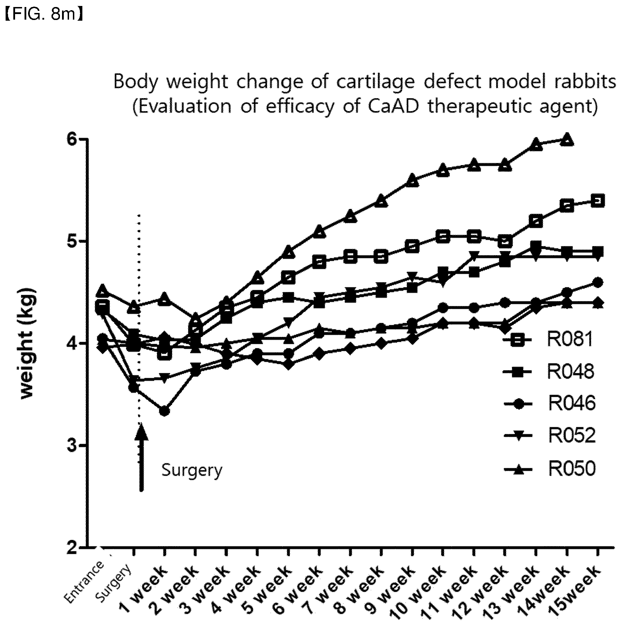

FIGS. 8a-8m show a result of implanting a spheroid to which an electric stimulus was applied into the femoral cartilage of the hind limb of an experimental animal (rabbit) and then investigating the therapeutic effect. FIG. 8a shows the overall process of the experiment, FIG. 8b and FIG. 8c show a result of extracting the femur 16 weeks after the implantation and imaging cartilage regeneration by micro-CT, FIGS. 8d-8g show a result of preparing histopathology slides and confirming histological regeneration through typical cartilage staining, FIGS. 8h-8l show a result of evaluating the regeneration of the spheroid to which the electric stimulus was applied for cartilage defects in five rabbits, and FIG. 8m shows a result of confirming that the experimental animal was not affected by inflammatory responses or health problems caused by the defects by measuring body weight.

### [Best Mode]

As a result of studies and efforts to develop a therapeutic agent that can treat a cartilage-related disease without addition of expensive growth factors, the inventors of the present disclosure have found out that stem cells can be differentiated into chondroprogenitor cells that have the characteristics of chondrocytes without expressing the mature chondrocyte marker COL2 by applying an electric stimulus and have completed the present disclosure.

Therefore, the present disclosure provides a pharmaceutical composition for treatment or prevention of a cartilage-related disease, which contains chondroprogenitor cells having the following characteristics or an aggregate thereof as an active ingredient:
(a) not expressing COL2; and (b) the chondroprogenitor cells being substrate-stained by one or more selected from a group consisting of alcian blue, safranin O and toluidine blue.

In the present disclosure, COL2 (collagen 2) forms the basis of articular cartilage and hyaline cartilage and accounts for 50% of total proteins in the cartilage and 85-90% of collagen in the articular cartilage. It is used as a typical marker of mature chondrocytes for this reason. Since the chondroprogenitor cells of the present disclosure do not express COL2 at all, it is confirmed that they are not mature chondrocytes.

In the present disclosure, the chondroprogenitor cells are prepared by applying an electric stimulus to stem cells, specifically mesenchymal stem cells, and are different from the mesenchymal stem cells themselves in that they do not express proliferation markers related with the sternness of the mesenchymal stem cells, such as MKI67, TOP2A and HMMR. The chondroprogenitor cells show characteristics similar to those of chondrocytes in that they can be substrate-stained with alcian blue, safranin O, etc. However, they are undifferentiated cells prior to differentiation into mature chondrocytes since they do not express COL2, which is a typical marker of mature chondrocytes, at all. They can be utilized as a formulation that can be easily implanted for treatment, etc. since they spontaneously aggregate into spheroids without external factors such as the addition of growth factors, centrifugation, etc.

In the present disclosure, the term "spheroid" refers to a cell aggregate in the form of a spheroid. It is generally produced by culturing in a 96-well plate, hanging drop, etc. For treatment of a cartilage-related disease, a process of implanting cells or an aggregate thereof is necessary. The spheroid form is suitable for the implantation process. In order to make the cell aggregate used in the implantation into the spheroid form, methods such as centrifugation, etc. are used. However, it was confirmed that the cell aggregate of the present disclosure forms a spheroid form that can be implanted easily only by applying an electric stimulus for inducing differentiation into chondroprogenitor cells without such additional steps.

The cartilage-related disease may be selected from a group consisting of osteoarthritis, arthritis, meniscus derangement, rheumatoid arthritis, tear of meniscus, triangular fibrocartilage complex tear, traumatic cartilage damage and degenerative arthritis, although not being limited thereto.

The term "prevention" refers to any action of suppressing symptoms or delaying the onset of a cartilage-related disease by administering the pharmaceutical composition according to the present disclosure.

The term "treatment" used in the present disclosure refers to any action of improving or favorably changing the symptoms of a cartilage-related disease by administering the pharmaceutical composition according to the present disclosure.

The active ingredient of the pharmaceutical composition may contain 90% or more, specifically 91%, 92%, 93%, 94%, 95%, 96%, 97%, 98%, 99% or more, of cells homogeneous with the chondroprogenitor cells. The cells may be those differentiated from stem cells. Specifically, the stem cells may be mesenchymal stem cells.

The mesenchymal stem cells (MSCs) of the present disclosure are cells that can differentiate into chondrocyte, osteocytes, adipocytes, myocytes, etc. and can be induced to differentiate into cartilage, bone, muscle, ligament and adipose tissues under specific culture conditions in vitro. Since the mesenchymal stem cells can be extracted easily from the bone marrow, many researches are being conducted on their potential for use as cell therapeutic agents for various intractable diseases. Since the cartilage lacks regenerative capacity, it is very difficult to be repaired once it is damaged. Since degenerative arthritis is caused by degenerative changes in the joint, it cannot be stopped completely. Currently, it is managed by drug therapy, physical therapy, etc. However, a definitive drug for treating arthritis has not been developed and long-term use of steroids and lubricants leads to accelerated degeneration of the cartilage. Although autologous chondrocyte transplantation has been developed recently, problems remain such as limitation in cartilage tissue, dedifferentiation of chondrocytes during in-vitro culture, limited cellular proliferation due to old age, etc. Therefore, the mesenchymal stem cells which have abundant regenerative ability can be used as an effective cell therapeutic agent for regenerating cartilage whose biological restoration has been destroyed. The cartilage regeneration using the cell therapeutic agent can be applied not only to musculoskeletal diseases but also to diseases of the digestive and urinary systems. That is to say, it can be used for treatment of diseases such as reflux esophagitis, urethral reflux, etc. since it locally regenerates the cartilage tissue.

The induction of differentiation in the present disclosure may be achieved by an electric stimulus. The, electric stimulus may have a frequency of 0 to 20 Hz, specifically 3 to 15 Hz or 5 to 12 Hz, although not being limited thereto. The electric stimulus may be a voltage with an amplitude of -20 V to 20 V, specifically -15 V to 15 V, more specifically -10 V to 10 V.

The chondroprogenitor cells of the present disclosure may have, a decreased expression level of one or more gene selected from a group consisting of COL1 and COL5 or a protein encoded by the gene as compared to mesenchymal stem cells; and an increased expression level of the COL6 gene or a protein encoded by the gene as compared to mesenchymal stem cells; and an increased expression level of one or more gene selected from a group consisting of GJB2, GJC1, PECAM1, CLDN2, CLDN7, CLDN10 and CLDN19 or a protein encoded by the gene as compared to mesenchymal stem cells, although not being limited thereto.

Through specific examples, the inventors of the present disclosure have confirmed that cells to which the electric stimulus of the present disclosure has been applied can differentiate into chondroprogenitor cells and they can be used to treat a cartilage-related disease.

In an example of the present disclosure, the inventors of the present disclosure have confirmed that cell aggregation occurs when an electric stimulus is applied to canine mesenchymal stem cells under a specific condition. It was confirmed that the cell aggregate differentiates into chondroprogenitor cells without addition of exogenous factors and they have the characteristics of chondrocytes since they can be substrate-stained by alcian blue and safranin O (see Example 2-1). It was confirmed that the cells prepared in this way show increased calcium oscillation during the differentiation as compared to the cells to which the electric stimulus was not applied. It was confirmed that cell aggregation occurs due to the electric stimulus and, through this, the mesenchymal stem cells differentiate into chondroprogenitor cells (see Example 2-2).

In other examples of the present disclosure, it was confirmed that the mesenchymal stem cells to which an electric stimulus of a specific condition was applied show no significant difference in viability despite the application of the electric stimulus (see Example 3-2) and the cells to which the electric stimulus was applied are different from mesenchymal stem cells since they do not express stem ness-related factors such as MKI67, TOP2A and HMMR (see Example 3-3) and are different from mature chondrocytes since they do not express COL2, which is a typical marker of mature chondrocytes, at all (see Example 3-4).

In another example of the present disclosure, it was confirmed that, when a defect was induced in a rabbit's cartilage area and chondroprogenitor cells prepared by applying an electric stimulus were implanted into the rabbit's cartilage defect area, the defective area could be regenerated to an effective level (see Example 5).

Through these results, the inventors of the present disclosure have confirmed that mesenchymal stem cells can be induced to differentiate into chondroprogenitor cells, which are different from mesenchymal stem cells or chondrocytes, by applying an electric stimulus of a specific condition even without addition of expensive growth factors. Since the chondroprogenitor cells of the present disclosure do not express COL2, which is a mature chondrocyte marker, at all, it is expected that they will be free from immune-related problems, which are main problems that occur during transplantation of mature cells, as immature cells.

In the present disclosure, the "pharmaceutical composition" refers to a composition prepared for the purpose of preventing or treating a disease and it can be formulated into various forms according to conventional methods. For example, it can be formulated into oral formulations such as a powder, a granule, a tablet, a capsule, a suspension, an emulsion, a syrup, etc. or into a formulation for external application, a suppository, a sterile injectable solution, etc.

The pharmaceutical composition according to the present disclosure may further contain a pharmaceutically acceptable carrier. The pharmaceutically acceptable carrier may be one commonly used in preparation and includes saline, sterile water, Ringer's solution, buffered saline, cyclodextrin, dextrose solution, maltodextrin solution, glycerol, ethanol, liposome, etc., although not being limited thereto. If necessary, the pharmaceutical composition may further contain other common additives such as an antioxidant, a buffer, etc. In addition, it may be formulated into an injectable formulation such as an aqueous solution, a suspension, an emulsion, etc., a pill, a capsule, a granule or a tablet by additionally adding a diluent, a dispersant, a surfactant, a binder, a lubricant, etc. Suitable pharmaceutically acceptable carriers and formulations are disclosed in Remington's Pharmaceutical Sciences (19th edition, 1995). The pharmaceutical composition of the present disclosure is not particularly limited in dosage form but may be formulated into an injection, an inhalant, a formulation for external application, an oral formulation, etc. It may be in a form that can be implanted easily, specifically a spheroid form. The spheroid may have a diameter of 0.5-1.5 mm, specifically 0.8-1.2 mm, more specifically 1.0 mm, although not being limited thereto.

The pharmaceutical composition of the present disclosure can be administered orally or parenterally (e.g., intravenously, subcutaneously, intraperitoneally or topically) depending on the intended method. The administration dosage may be determined adequately by those skilled in the art although it varies depending on the patient's condition and body weight, the severity of a disease, drug type, administration route and administration time.

The pharmaceutical composition of the present disclosure is administered in a pharmaceutically effective amount. In the present disclosure, the "pharmaceutically effective amount" refers to an amount sufficient to treat a disease with a reasonable benefit/risk ratio applicable to medical treatment. The effective dosage level may be determined in consideration of the type and severity of a disease, the activity of a drug, the sensitivity to the drug, administration time, administration route, excretion rate, treatment period, drugs used concurrently and other factors well known in the medical field.

The pharmaceutical composition according to the present disclosure may be administered as an individual therapeutic agent or together with another therapeutic agent. It may be administered sequentially or simultaneously with a conventional therapeutic agent. It is important to administer a minimum amount that can achieve maximum effect without side effects in consideration of all the factors described above, and it can be easily determined by those skilled in the art.

The present disclosure also provides a method for prevention or treatment of a cartilage-related disease, which includes a step of administering the pharmaceutical composition to a subject.

The term "administration" used in the present disclosure refers to provision of the composition of the present disclosure to a subject by any suitable method.

The term "subject" used in the present disclosure refers to a subject in need of treatment of a disease. More specifically, it refers to a mammal such as human, a non-human primate, mouse, dog, cat, horse, cow, etc.

The present disclosure also provides a use of the pharmaceutical composition for prevention or treatment of a cartilage-related disease.

The present disclosure also provides a method for preparing a pharmaceutical composition for treatment or prevention of a cartilage-related disease, which includes a step of preparing chondroprogenitor cells or an aggregate thereof by applying an electric stimulus to stem cells, wherein the chondroprogenitor cells have the following characteristics: (a) not expressing COL2; and (b) the chondroprogenitor cells being substrate-stained by one or more selected from a group consisting of alcian blue, safranin O and toluidine blue.

Hereinafter, specific examples are presented to help the understanding of the present disclosure. However, the following examples are provided only to make the present disclosure easier to understand and the contents of the present disclosure are not limited by the examples.

### [Examples]

### Example 1. Experimental materials and experimental methods

### 1-1. Primary cell culturing

3 batches of canine adipose-derived stem cells (hereinafter, referred to as ADSCs) were acquired from the abdominal adipose tissues of three 4-month-old female beagle dogs. The cells were stored at passage 0 and their ability to differentiate into adipocytes, chondrocytes and osteoblasts was evaluated. The cells were plated in a 75-cm² flask (5×10⁵ cells/flask, BD Falcon) and cultured in lowglucose DMEM (Dulbecco's modified Eagle's medium, GIBCO) containing 10% FBS (GIBCO) and 1x antibiotic-antimycotic (GIBCO).

### 1-2. Culturing of micromass and electric stimulation

After detaching the canine ADSCs at passages 3-5, they were suspended at a high density (2.5×10⁷ cells/mL) in serum-free advanced DMEM/F12 medium (GIBCO) containing 1x antibiotic-antimycotic and 1x GlutaMax (GIBCO). To generate a micromass, 10 µL of the cell suspension was placed on a 35-mm² dish (Corning) and allowed to attach in an incubator (N-Biotek, Korea) under the condition of 37 ºC and 5% CO₂. After 1 hour of incubation, serum-free advanced DMEM/F12 medium (GIBCO) was added. The cells were placed in a multi-channel stimulator that can apply chronic stimulation to cells. The micromass of the canine primary ADSCs was cultured in the presence or absence of electric stimulation (hereinafter, referred to as ES) with 10 V/cm for 10 ms at a frequency of 2.0 Hz. After applying the electric stimulus for 3 days, the condensed cell mass was observed using a phase-contrast microscope (Eclipse Ti2, Nikon, Japan).

### 1-3. Single cell preparation and RNA sequencing

Single cell preparation was performed at room temperature using a MACS tissue dissociation kit (Miltenyi Biotech)) and a gentleMACS dissociator (Miltenyi Biotech). The cell suspension was transferred to a cell strainer and washed with a complete medium. Live/Dead viability assay (Molecular Probe) was performed to determine cell viability (live staining: > 90% cell viability as cut off). The RNA-seq analysis service (Macrogen, Korea) was used for RNA sequencing.

For preparation of a single-cell library, the cells were applied to the 10x Genomics platform using Next GEM Single Cell 3' Library & Single Cell3' V3.1 gel beads following the manufacturer's instructions that allow to capture approximately 500 cells. A uniquely barcoded cDNA library was obtained from RNA, which was reverse-transcribed within single-cell droplets, purified and enriched through PCR. The library was sequenced using HiSeqX (Illumina) with a read length of 28 bp for read 1 (cell barcode and unique molecular identifier [UMI]), 8 bp for index read (sample barcode) and 91 bp for read 2 (RNA read).

For data analysis, Cell Ranger v3.1.0 (10x Genomics) was used to generate FASTQ files. For this, the data were aligned with the canine reference genome (CanFam3.1 release 100) and the expression of genes was measured with UMI and cell barcode. Differential gene expression analysis was performed after the cell cluster was determined. The final aggregation dataset was imported to Seurat 3.1.3 for normalization with multiple datasets. Cells with mitochondria percentage of > 0.2 were filtered to exclude the cells with a low UMI content. UMAP (uniform manifold approximation and projection) analysis was performed on the basis of the statistically significant principal components. The significant markers for every cluster compared with all remaining cells were determined using the minimum fraction of minimum percentage of cells and a Wilcox rank-sum test was performed. Only the significant results were reported.

### 1-4. Genomic analysis using online database

To assess the chondrogenic differentiation of canine ADSCs, transcriptome changes were analyzed using NCBI GEO (Gene Expression Omnibus). For this, GSE32398 (top 250 genes modified in human articular cartilage compared with the growth plate cartilage), GSE51812 (top 239 genes modified in human articular chondrocytes at week 17 of development compared with prechondrocytes at week 6 of embryogenesis) and GSE19664 (top 128 genes modified in healthy human BMSCs (bone marrow-derived stem cells) over time during chondrogenesis) transcriptome datasets were used. GO (Gene Ontology) annotation and PANTHER classification system were used to analyze the significant probe lists.

### 1-5. Measurement of calcium oscillation

Canine primary ADSCs were suspended at a density of 2-2.5×10⁷ cells/mL and a 10 µL drop of the cell suspension was placed on a CellBIND surface 35-mm² dish (Corning). The micromass of the canine ADSCs was cultured in the presence or absence of electric stimulation (10 V/cm for 10 ms at a frequency of 2.0 Hz). After 14 hours ES, a Fluo-4 reagent (Molecular Probe) without probenecid was loaded in the medium at 1:1 ratio for 30 minutes according to the manufacturer's protocol. Calcium oscillation was recorded at 1 fps after excitation at 488 nm with 1 second exposure time during 10 minutes without incubation. Time-lapse changes in fluorescence intensity were analyzed using the NIS-Elements Advanced Research Imaging software (Eclipse Ti2, Nikon, Japan).

### 1-6. Measurement of total glycosaminoglycan

For measurement of glycosaminoglycan (hereinafter, referred to as GAG), cells were washed with PBS, fixed with paraformaldehyde (Biosesang) for 20 minutes and then stored at 4 ºC before staining. The cells were incubated for 30 minutes at room temperature with an alcian blue (IHC World) solution or for 1 hour at room temperature with a safranin O (Safranin-O, IHC World) solution and then rinsed several times with distilled water. The accumulation of GAG was imaged using a digital USB camera (My First Lab, USA).

### 1-7. Flow cytometry

The single cells prepared in Example 1-3 were stained by treating with Alexa488 anti-dog CD44 (MCA1041A488, Bio-Rad), PE anti-dog CD90 (12-5900-42, BD), PerCP-Cy5.5 anti-dog CD29 (303024, BioLegend), Alexa488 anti-dog CD45 (MCA1042F, Bio-Rad), Alexa647 anti-dog CD73 (Bs-4834R-A647, Bioss), FITC anti-dog CD54 (GTX76274, GeneTex), APC anti-dog CD49d (304308, BioLegend), PE anti-dog CD34 (559369, BD), PE anti-hu/dog HLA-DR (361606, BioLegend) or APC anti-dog CD80 (104714, BD) at 4 ºC for 20 minutes. Flow cytometric measurements were conducted using the BD LSRII analysis service at Yonsei University Clinical and Academic Research Institute.

### 1-8. RT qPCR analysis

Total RNA was isolated from canine ADSCs cultured under various conditions for 3 days using a GentleMACS dissociator (Miltenyi Biotech) and Direct-zol RNA MiniPrep (Zymo Research) following the manufacturer's protocol. RNA concentration was measured using a biospectrometer (Eppendorf) and reverse transcription reaction was performed with 0.3-0.5 µg of total RNA using a TOPscript cDNA synthesis kit (Enzynomics). Real-time PCRs for GAPDH and COL1A1 were conducted using SYBR 2x Mix (Bio-Rad) with 10 ng cDNA/tube and a CFX Connect real-time PCR detection system (Bio-Rad). Specifically, the samples were held at 95 ºC for 15 minutes followed by 40 amplification cycles including denaturation at 95 ºC for 10 seconds and extension and annealing at 60 ºC for 30 seconds.

The expression level of COL1A1 was normalized to the expression level of GAPDH and the relative gene expression level was calculated using the 2-ΔΔCT method. Primer sequences were self-determined using Primer-BLAST and were as follows: canine GAPDH forward primer 5'-GGTGATGCTGGTGCTGAGTA, reverse primer 5'-GGCATTGCTGACAATTCTGA; canine COL1A1 forward primer 5'-CCGCTTCACCTACAGTGTCA, reverse primer 5'-CAGACAGGGCCAATATCCAT(Bioneer, Korea).

### 1-9. Western blot analysis

The cells were washed 3 times with ice-cold PBS and harvested in a RIPA cell lysis buffer (GenDEPOT) containing phosphatase inhibitor cocktails (GenDEPOT). The protein concentration was determined using a BCA protein assay kit (Pierce). Protein samples were separated on SDS-PAGE gel and electrotransferred to a PVDF membrane (ATTO) using standard procedures. The membrane was blocked with 5% nonfat dry milk dissolved in 0.05% TBST and then incubated with primary antibodies at ºC for 12 hours on a rocking platform. The membrane was then washed 3 times with a TBST buffer for 15 minutes and incubated with 1% skim milk in TBST containing HRP-conjugated secondary antibodies (GeneTex) for 1 hour. The hybridized membrane was washed with a TBST buffer and visualized using an enhanced chemiluminescence detection kit (Merck) and a LAS500 imaging system (GE Healthcare.

### 1-10. Karyotyping

For sampling, the cellular condensations induced by ES were disrupted into a uniform single-cell suspension using a nylon mesh with a pore size of 70 µm. The cell suspension was collected in a 15-mL tube and then centrifuged at 450 × g for 5 minutes. After aspiration of the medium, the pellet was suspended in a fresh medium and karyotyping was analyzed using G-banding staining and a chromosome imaging analyzer system at the GenDix Karyotyping Service.

### 1-11. Statistical analysis

All data were presented as mean ± standard deviation (n = number of individual samples). All statistical analyses were performed using the MS Excel software (Microsoft 365) and P values of < 0.05 were considered to indicate significant difference.

### Example 2. Preparation of chondroprogenitor cells through application of electric stimulus to stem cells

### 2-1. Chondrogenesis of ADSCs by application of electric stimulus

After applying ES (10 V/cm, 10 ms, 2 Hz frequency) to canine ADSCs (MSCs), aggregate formation was assessed. The micromass of the canine ADSCs was performed without addition of serum and exogenous factors (FIG. 1a). After 3 days of ES application, the result of immunohistochemical staining of cartilaginous matrixassociated molecules of the aggregate was investigated by phase-contrast microscopy. As shown in FIG. 1b, the formation of sheet-like aggregates and larger condensation were observed in the micromass subjected to ES compared with those without ES. Furthermore, more intense alcian blue and safranin O staining was observed in the cells subjected to ES, suggesting the deposition of proteoglycan (FIG. 1c).

In order to investigate the effect of ES on cell death, cell viability was assessed using a Live/Dead reagent. As shown in FIG. 1d, the application of ES did not result in significant difference in cell viability. As shown in FIG. 1e, the aggregate contained approximately 10⁵ cells. In order to investigate the change in cell membrane proteins caused by the ES application, flow cytometry analysis was performed using antibodies against surface molecules. The cell masses showed a similar expression pattern of CD44, CD90, CD29, CD73, CD54, CD34, CD49d, CD45, HLA-DR and CD80, compared with ADSCs growing in monolayer (FIG. 1f).

Through these results, it was confirmed that the ES induces canine ADSCs to produce highly compact prechondrogenic condensation.

### 2-2. Confirmation of calcium oscillation during chondrogenesis

It was previously reported that chondrogenesis driven by ES reproduced the spontaneous intracellular calcium oscillation observed in cartilage development. It was monitored whether similar calcium oscillation occurs also in the micromass of canine ADSCs in the presence of ES. The fluorescence measurement with Fluo-4 fluctuated at high amplitude with a more regular frequency in the cell mass subjected to ES than in the control mass (FIG. 2a). More specifically, as shown in FIG. 2b and FIG. 2c, the electrically stimulated cell aggregate showed a typical pattern of calcium oscillation. This result suggests that prechondrogenic condensation is induced in the ADSCs during aggregation of the micromass in the presence of ES without exogenous factors.

### Example 3. Characterization of chondroprogenitor cells prepared by electric stimulation

### 3-1. Confirmation of transcription profile

ES Single cells were isolated from the electrically stimulated cell mass. The single-cell RNA-seq library was prepared on the 10x Genomics Chromium platform and data were imported into standard Seurat, a toolkit for analysis. The overall experiment for confirmation of the cellular transcription profile is shown in FIG. 3a. Specifically, highly variable genes were identified by analyzing the normalized dataset using Loupe Cell Browser and 12 clusters were subdivided into four datasets of (FIG. 3b). Also, as shown in FIG. 3c, significant changes in the transcriptome of the cell micromass subjected to ES from the 2D ADSCs in terms of phenotypes and functions.

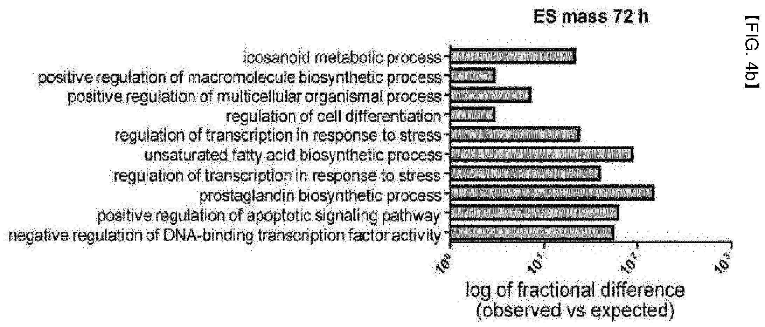

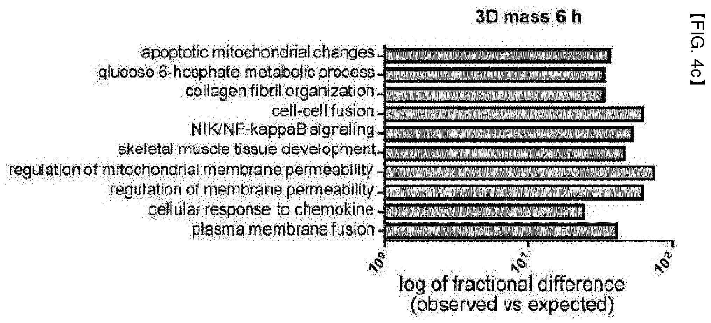

The gene expression patterns of the four datasets were explored using open-source GO annotation and the PANTHER database analysis tool. It was confirmed that the genes associated with proton ion transport, ornithine metabolism, biogenesis and calcium ion import/release were upregulated in the ADSCs micromass subjected to ES for 6 hours (FIG. 4a). In the cell micromass without ES, it was confirmed that the genes associated with cartilage condensation, calcium ion transmembrane transport, skeletal muscle tissue development, NF-κB pathway and chemotaxis of immune cells were upregulated (FIG. 4b). It was confirmed that the application of ES to the cell micromass for 72 hours induced the upregulation of genes associated with prostaglandin synthesis, multicellular organism development, arginine catabolism and negative regulation of apoptosis (FIG. 4c).

The gene expression patterns were compared with publicly available datasets of gene expression to understand the role in cartilage development. As shown in FIGS. 4d-f, it was confirmed that the application of ES to the cell micromass for 72 hours induced prechondrogenic changes in considerable number of genes that were upregulated during the articular chondrocyte differentiation from the embryonic chondrogenic condensation. In a research conducted to identify the key molecules involved in the differentiation of osteochondral progenitor cells, it was confirmed that the balance between RUNX2 and SOX9 in the osteochondral progenitor cells plays an essential role in the differentiation of chondrocytes and osteoblasts. The present data for the micromass subjected to ES show that the levels of RUNX2 and its repressor, NKX3.2, were specifically altered to amounts typical for prechondrogenic cells (FIG. 4g).

Through these results, it was confirmed that the condensation of canine ADSCs triggered by ES induced a phenotype similar to the chondroblast developmental stage.

### 3-2. Confirmation of viability

Since physiological stresses caused by ES might affect several cellular processes and might lead to diverse physiological and pathological outcomes, cell viability was assessed after 3 days of ES application to ADSCs using a CCK-8 viability kit. As shown in FIG. 5a, the application of ES had no effect on the cell viability of the micromass of canine ADSCs. The analysis of the GO database indicated that the electrical exposure did not affect the expression of SHARPIN, which is an apoptosisassociated gene (FIG. 5b). A single ADSC suspension prepared by mechanical and enzymatic dissociation of the cell micromass was stained with PI (propidium iodide). As shown in FIG. 5c, the number of PI-stained cells was irrelevant of the ES application.

### 3-3. Confirmation of differentiation into chondroprogenitor cells

Adult stem cells express representative proliferation markers such as MKI67, TOP2A and HMMR as compared to growing normal cells. As shown in FIG. 5d, it was confirmed that the proliferation markers were not expressed in the aggregated cells regardless of the ES application. Furthermore, no change in karyotype was observed in the cells subjected to ES (FIG. 5e).

It was confirmed that the cell aggregate differentiated into mature cells once they were aggregated.

### 3-4. Confirmation of change in collagen production

The main function of chondrocytes is to synthesize extracellular matrix components such as collagen types 2, 4, 6, 10, 11, 12 and 14. Therefore, the level of collagen production was investigated in the cells subjected to ES. As shown in, FIG. 6a, the expression of COL3A1, COL4A1, COL4A2, COL4A5, COL5A3, COL6A1, COL6A3, COL6A5, COL8A1, COL13A1, COL14A1, COL15A1, COL16A1, COL21A1, COL23A1, COL24A1, COL24A1 and COL27A1 was increased in the micromass of canine ADSCs regardless of the ES application. Moreover, the expression of COL3A1, COL5A2 and COL6A1/3 increased strongly and uniformly in the micromass of ADSCs. The expression of COL6A3 and COL16A1 was suppressed in the micromass as compared to the cells not subjected to ES.

However, COL2, which is expressed during the chondrogenesis of MSCs and known as a marker of mature chondrocytes, was not expressed in the presence or absence of ES (FIG. 6b). It was confirmed in the comparative examples of a previously filed patent (KR 10-2015-0047361), which discloses a method of differentiating stem cells into chondrocytes by applying an electric stimulus to mesenchymal stem cells, that the expression of COL2, which is used as a mature chondrocyte marker, was increased significantly both in mesenchymal stem cells to which growth factors were added and in mesenchymal stem cells to which the electric stimulus was applied. However, in the present disclosure, it was confirmed that the cell aggregate to which the electric stimulus was applied do not express COL2 at all.

RT-qPCR (real-time quantitative PCR) was performed to investigate the effect of ES on type 1 collagen. As shown in FIG. 6c, it was confirmed that the expression of COL1A1 was increased significantly in the cells subjected to ES as compared to the cells not subjected to ES. A similar result was obtained by western blotting for the COL1A1 (FIG. 6d).

### 3-5. Confirmation of expression of factors related to collagen regulation depending on application of electric stimulus

The mechanism of the change of the expression level of type 1 collagen in response to electric stimulation was analyzed. For this, the open-source database provided by GeneHancer, which integrates enhancers from various sources, was utilized. It was confirmed that the levels of the transcription factors NR4A1, RBFOX2, NFIC, ID3, HDGF, BMI1, YBX1, SMARCE1, HLTF and SMC3, which are known to be associated with the expression of COL1A1 in the cells to which ES was applied, decreased significantly over time (FIG. 7a). On the contrary, the transcription factors such as TCF12, POLR2A, ATF4, ARID4B, SMARCA5, GTF2F1, LARP7, HDAC2 and YY1 increased in the cell micromass for 72 hours after the application of ES (FIG. 7b).

These factors are associated with TGFβ signaling and, thus, cause change in the expression level of COL1A1. Despite the increase in the expression of the transcription factors associated with COL1A1, the expression of COL1A1 decreased significantly. This result suggests that the expression of COL1A1 is affected by factors other than the transcription factors.

### Example 4. Confirmation of tissue formation ability of chondroproqenitor cells

In order to ascertain the role of ES on the cytoskeleton, the expression of genes which specifically regulate the cytoskeleton organization including cell-cell junction was investigated. As shown in FIGS. 7d and 7e, it was confirmed that the expression of connexins (GJB2/GJC1), cell junction proteins (PECAM1) and claudins (CLDN2/CLDN7/CLDN10/CLDN19)), which have been reported to be functionally essential for the cartilage extracellular matrix, was significantly upregulated.

This result is consistent with the GAG staining result of Example 2-1. ES application to the cell micromass induced significantly higher expression of a gene associated with the biosynthesis of chondroitin sulfate and keratan sulfate, which are two GAGs that constitute aggrecan. These data confirm that the electric stimulus can directly induce differentiation into chondroprogenitor cells and can repair hyaline cartilage by stimulating the plasticity of the cartilage matrix and chondroprogenitor cells.

### Example 5. Confirmation of therapeutic effect of chondroprogenitor cells in vivo

To confirm the histological regeneration and repair of cartilage in vivo depending on the differentiation of a prechondrogenic cell aggregate to which an electric stimulus was applied to cartilage tissue and tissue engraftment, a small cartilage area with a diameter of 4 mm (rabbit's average cartilage thickness: 0.3 mm) in the femoral cartilage of an experimental New Zealand white rabbit (NZW rabbit) which weighed 4 kg or more was created. Eight electrically stimulated aggregates were implanted and fixed by applying fibrin glue. The overall experimental procedure is shown in FIG. 8a. Since the implanted electrically stimulated aggregates were derived from adipose-derived mesenchymal stem cells, cyclosporine, an immunosuppressant, was administered intravenously once a day (10 mg/kg) in consideration of xenograft transplantation. The experiment was stopped immediately when normal health condition was not shown within 2 weeks. At 16 weeks after the implantation, the femoral cartilage was extracted by performing an autopsy and the defect and regeneration of the femoral cartilage were evaluated with images obtained by imaging with micro-CT having a micrometer-scale resolution. As shown in FIG. 8b and FIG. 8c, it was confirmed that the defect area where the electrically stimulated aggregates were implanted was recovered 4 months later to a level evaluated as the cartilage layer.

Cartilage matrix staining was performed to confirm the above result. As shown in FIGS. 8c-8g, differentiation into chondrocytes and recovery of the cartilage matrix as in normal cartilage tissue were confirmed through histological evaluation of the implanted electrically stimulated aggregates through cartilage matrix staining with alcian blue, trichrome and safranin O as compared to the defect. In addition, long-term engraftment in vivo, connection with surrounding normal cartilage and smooth tissue adhesion with the underlying bone tissue were confirmed. These results are consistent with the histopathological staining result confirmed above.

Significant regeneration and recovery were confirmed for the five rabbits (FIGS. 8h-8m). No inflammation was observed in the small cartilage area or the implanted aggregate area of the experimental animal. No abnormality was observed in health monitoring of the experimental animal through body weight measurement and visual inspection, and no additional effects were considered when considering the effectiveness of the implanted aggregates.

The foregoing description of the present disclosure is provided only for illustration. Those having ordinary knowledge in the art to which the present disclosure belongs will understand that the technical idea or essential features of the present disclosure can be modified easily. Therefore, the exemplary embodiments described above should be understood as illustrative, not limitative.

## Claims

1. A pharmaceutical composition for treatment or prevention of a cartilage-related disease, comprising chondroprogenitor cells having the following characteristics or an aggregate thereof as an active ingredient:
(a) not expressing COL2; and
(b) the chondroprogenitor cells being substrate-stained by one or more selected from a group consisting of alcian blue, safranin O and toluidine blue.

2. The pharmaceutical composition according to claim 1, wherein the cartilage-related disease is selected from a group consisting of osteoarthritis, arthritis, meniscus derangement, rheumatoid arthritis, tear of meniscus, triangular fibrocartilage complex tear, traumatic cartilage damage and degenerative arthritis.

3. The pharmaceutical composition according to claim 1, wherein the active ingredient of the pharmaceutical composition comprises 90% or more cells homogeneous with the chondroprogenitor cells.

4. The pharmaceutical composition according to claim 1, wherein the chondroprogenitor cells are induced to differentiate from stem cells.

5. The pharmaceutical composition according to claim 4, wherein the stem cells are mesenchymal stem cells.

6. The pharmaceutical composition according to claim 4, wherein the induction of differentiation is achieved by an electric stimulus.

7. The pharmaceutical composition according to claim 6, wherein the electric stimulus has
a frequency of 0 to 20 Hz;
an amplitude of -20 to 20 V; and
a duty ratio of 0 to 80%.

8. The pharmaceutical composition according to claim 1, wherein the chondroprogenitor cells have a decreased expression level of one or more gene selected from a group consisting of COL1 and COL5 or a protein encoded by the gene as compared to mesenchymal stem cells; and have an increased expression level of the COL6 gene or a protein encoded by the gene as compared to mesenchymal stem cells.

9. The pharmaceutical composition according to claim 1, wherein the chondroprogenitor cells have an increased expression level of one or more gene selected from a group consisting of GJB2, GJC1, PECAM1, CLDN2, CLDN7, CLDN10 and CLDN19 or a protein encoded by the gene as compared to mesenchymal stem cells.

10. The pharmaceutical composition according to claim 1, wherein the pharmaceutical composition is in a dosage form that is easy to be inserted directly into the cartilage.

11. The pharmaceutical composition according to claim 1, wherein the aggregate of the chondroprogenitor cells is in a spheroid form.

12. The pharmaceutical composition according to claim 8, wherein the spheroid has a diameter of 0.5-1.5 mm.

13. A method for preparing a pharmaceutical composition for treatment or prevention of a cartilage-related disease, comprising a step of preparing chondroprogenitor cells or an aggregate thereof by applying an electric stimulus to stem cells, wherein the chondroprogenitor cells having the following characteristics:
(a) not expressing COL2; and
(b) the chondroprogenitor cells being substrate-stained by one or more selected from a group consisting of alcian blue, safranin O and toluidine blue.

14. A method for treatment or prevention of a cartilage-related disease, comprising a step of administering chondroprogenitor cells having the following characteristics or an aggregate thereof to a subject in need thereof:
(a) not expressing COL2; and
(b) the chondroprogenitor cells being substrate-stained by one or more selected from a group consisting of alcian blue, safranin O and toluidine blue.

15. A use of chondroprogenitor cells having the following characteristics or an aggregate thereof for preparing a medicine for treatment or prevention of a cartilage-related disease:
(a) not expressing COL2; and
(b) the chondroprogenitor cells being substrate-stained by one or more selected from a group consisting of alcian blue, safranin O and toluidine blue.
